# FASCICULE DE BREVET EUROPEEN

(11) **EP 2 568 974 B1**
(45) Date de publication et mention de la délivrance du brevet: **04.01.2017**
(21) Numéro de dépôt: 11714022.8
(22) Date de dépôt: 07.04.2011
(51) Int. Cl.: A61K 9/50, A61K 31/485

(54) **FORME PHARMACEUTIQUE ORALE ALCOOLO-RESISTANTE**
ALKOHOLRESISTENTE ORALE PHARMAZEUTISCHE FORM
ALCOHOL-RESISTANT ORAL PHARMACEUTICAL FORM

(30) Priorité: 14.05.2010 FR 1053763
(43) Date de publication de la demande: 20.03.2013
(73) Titulaire: Ethypharm, 92210 Saint-Cloud (FR)
(72) Inventeur: HERRY, Catherine, F-27670 Saint-Ouen du Tilleul (FR); TRICHARD, Laury, F-27340 Pont de l'Arche (FR)
(74) Mandataire: Regimbeau
(86) Numéro de dépôt international: PCT/EP2011/055460
(87) Numéro de publication internationale: WO 2011/141241

(56) Documents cités:
- WO-A1-2009/036811
- WO-A2-2006/125819
- WO-A2-2008/021394
- WO-A2-2010/037854
- US-A1- 2005 020 613
- US-A1- 2007 264 346
- US-A1- 2008 069 891
- US-A1- 2009 041 838

## Description

L'invention a pour objet une nouvelle forme pharmaceutique orale à base de microgranules, cette forme étant à libération prolongée d'au moins un principe actif et dont la cinétique de libération permet notamment une prise journalière unique par le patient tout en évitant que cette libération soit accélérée par une prise concomitante d'alcool.

De nombreuses formes pharmaceutiques orales à libération prolongée existent sur le marché. La libération du principe actif doit être contrôlée en fonction de l'objectif thérapeutique et des propriétés pharmacologiques du principe actif. Certains principes actifs peuvent se révéler très toxiques, voire mortels, si la dose ingérée dépasse un certain seuil.

Il est donc impératif que leurs propriétés "retard" soient étroitement contrôlées, et ceci afin de s'assurer qu'une libération rapide du principe actif, ou "dose- dumping", ne puisse pas se produire, notamment lors d'une prise concomitante d'alcool. La consommation d'alcool en même temps qu'une prise de médicament peut en effet altérer la forme pharmaceutique qui libère alors très rapidement la totalité du principe actif qu'elle contient. Par ailleurs, pour améliorer le confort des patients, il est souhaitable de proposer un médicament présentant un effet pharmacologique sur une longue période de temps après administration.

Par exemple, ceci est particulièrement vrai pour les personnes souffrant de douleurs sévères et pour lesquelles la réponse pharmacologique doit être maintenue sur une longue période à un niveau thérapeutique constant au cours du temps.

Afin d'évaluer la résistance à l'alcool des compositions pharmaceutiques, la FDA (Food And Drug Administration) suggère la réalisation de tests de dissolution in vitro pour comparer les cinétiques obtenues dans le milieu HCl 0,1 N (représentatif du pH gastrique) avec les cinétiques obtenues dans le même milieu substitué à 5, 20 et 40% (v/v) par de l'éthanol. D'après Walden et al. (The Effect of Ethanol on the Release of Opioids from Oral Prolonged-Release Préparations Drug Development and Industrial Pharmacy, 33:10, 1101 - 1111 , 2007), le fait d'exposer in vitro une forme pharmaceutique sur une période de 2h est considérée comme représentative de la durée d'exposition de ces formes pharmaceutiques in vivo.

Il est connu de l'état de la technique le brevet EP1 189 602 qui décrit des microgranules de Sulfate de Morphine à libération prolongée. Ce document propose des microgranules constitués d'un support neutre enrobé d'une couche active et d'une couche à libération prolongée contenant un copolymère d'acide méthacrylique et d'ester méthyl méthacrylate ainsi qu'une silice hydrophobe. Cependant, cette forme de microgranule présente le désavantage de libérer très rapidement le principe actif en présence d'alcool ce qui peut être dommageable, voire létal pour le patient.

Il est également connu de l'état de la technique la demande de brevet WO2010037854 qui propose d'utiliser un support de neutres insolubles ou rendus insolubles dans l'eau ou dans l'alcool pour éviter une décharge immédiate du principe actif induite par la consommation d'alcool. Cependant, ce support est limité quant à sa résistance à l'alcool puisque le pourcentage maximum d'éthanol dans le milieu de dissolution est de 30%, ainsi ce support ne résisterait pas à des volumes importants d'alcools forts comme le Whisky ou la Vodka. De plus, cette demande de brevet ne permet pas d'obtenir une cinétique de dissolution compatible avec une administration unique journalière.

Les demandes WO2009/036812 , WO2009/0368811 et WO2010/034342 quant à elles portent sur des compositions pharmaceutiques à libération contrôlée pH-dépendantes comprenant des opioïdes et dont la cinétique de dissolution n'est pas sensiblement affectée par la présence d'éthanol. Plus particulièrement, ces compositions pharmaceutiques sont essentiellement constituées d'un coeur comprenant la substance active sur lequel est enrobée une couche d'un mélange de polymère et d'un mélange de lubrifiant inerte.

Cependant, la description contraint d'utiliser des polymères pH-dépendants qui peuvent induire des variabilités de comportement chez l'homme en fonction des individus et des conditions de prise de médicament (avec ou sans nourriture notamment).

Un objectif essentiel de la présente invention est donc de proposer une forme pharmaceutique orale à base de microgranules à libération prolongée d'au moins un principe actif, permettant d'éviter ou de limiter une décharge immédiate du principe actif induite par la consommation d'alcool lors de l'administration de cette forme pharmaceutique. Par ailleurs, cette forme pharmaceutique doit être facile à produire industriellement et à moindre coût.

Définitions au sens du présent exposé de l'invention :

### Support neutre

On entend par "support neutre" ou "noyau neutre" ou encore plus simplement "neutre", des supports inertes sphériques ou quasi-sphériques de taille comprise entre 50 µm et 3 mm et préférentiellement entre 100 et 1000 µm tels que ceux habituellement utilisés dans l'industrie pharmaceutique comme support de base de principes actifs pour la constitution de microgranules par exemple.

### Microgranules

Les microgranules de la présente invention se rapportent à des unités galéniques sphériques, constituées en leur centre d'un support neutre, recouvert d'au moins une couche contenant le principe actif qui est elle-même recouverte d'au moins une couche polymérique.

### Forme pharmaceutique

On entend par « forme pharmaceutique orale » toute forme pharmaceutique orale susceptible d'être préparée à partir de microgranules comprenant le principe actif, notamment une suspension, un sirop, un comprimé, une gélule, sachet.

### Libération prolongée

Dans la présente demande, on utilisera le terme libération prolongée pour désigner un profil de libération du principe actif modifié par rapport à celui qu'aurait présenté le principe actif seul dans un système à libération immédiate comme défini par la Pharmacopée Européenne (quantité de principe actif libérée en 45 minutes au moins égale à 75%, Ph. Eur., 6^{ème} édition 2.9.3.)

### Alcool

Le terme "alcool" représente l'éthanol et les termes "solution alcoolique" ou "milieu alcoolique" représente une solution aqueuse d'éthanol.

Le but de la présente invention est d'offrir une nouvelle composition pharmaceutique orale résistante à la décharge immédiate de la dose de principe actif due à l'alcool et permettant notamment une prise journalière unique.

Préférentiellement, on entend par « résistance à la décharge immédiate de la dose de principe actif due à l'alcool » ou bien « résistance à l'alcool » que le pourcentage d'actif libéré après 2h dans un milieu acido-alcoolique HCl 0,1 N contenant de l'alcool et de préférence une quantité d'éthanol comprise entre 4 et 40% (par exemple 10, 20, 30 ou 40%), n'est pas supérieur de plus de 15 points (15% en valeur absolue) comparé à celui libéré dans un milieu d'acide HCl 0,1 N.

### DESCRIPTION DETAILLEE DE L'INVENTION

Un microgranule selon l'invention comprend du centre vers la périphérie
- un support neutre,
- au moins une couche de montage comprenant au moins un principe actif et un agent liant pharmaceutiquement acceptable,
- au moins une couche d'enrobage comprenant
   - un polymère d'enrobage hydrophobe choisi parmi les dérivés non hydrosolubles de la cellulose, et
   - au moins 20 % de talc comme charge inerte par rapport à la masse sèche du polymère d'enrobage hydrophobe.

Le polymère hydrophobe empêche la libération immédiate du principe actif.

L'enrobage assure une libération prolongée du principe actif selon une cinétique de libération adaptée notamment à une prise journalière unique

L'invention a ainsi pour objet des microgranules à libération prolongée comprenant du centre vers la périphérie :
- un support neutre,
- au moins une couche de montage comprenant au moins un principe actif et un agent liant pharmaceutiquement acceptable;
- au moins une couche d'enrobage à libération prolongée comprenant :
   - un polymère d'enrobage hydrophobe choisi parmi les dérivés non hydrosolubles de la cellulose;
   - au moins 20% de talc comme à charge inerte par rapport à la masse sèche du polymère d'enrobage hydrophobe

Les microgranules selon l'invention sont notamment aptes à être administrés oralement en dose unique journalière.

Les microgranules selon l'invention présentent une résistance à l'alcool selon laquelle le pourcentage d'actif libéré après 2h dans un milieu acido-alcoolique HCl 0,1 N contenant de l'alcool et de préférence une quantité d'éthanol comprise entre 4 et 40% (par exemple 10, 20, 30 ou 40%), n'est pas supérieur de plus de 15 points (15% en valeur absolue) comparé à celui libéré dans un milieu d'acide HCl 0,1 N.

L'invention a également pour objet l'utilisation d'une charge inerte dans l'enrobage de microgranules à libération prolongée comprenant du centre vers la périphérie :
- un support neutre,
- au moins une couche de montage comprenant au moins un principe actif et un agent liant pharmaceutiquement acceptable;
- au moins une couche d'enrobage à libération prolongée comprenant un polymère d'enrobage hydrophobe choisi parmi les dérivés non hydrosolubles de la cellulose,
où la quantité de charge inerte dans l'enrobage à libération prolongée des microgranules est d'au moins 20 % de talc par rapport à la masse sèche du polymère d'enrobage hydrophobe,
pour conférer auxdits microgranules une résistance à l'alcool.

La résistance à l'alcool signifie que le pourcentage d'actif libéré après 2h dans un milieu acido-alcoolique HCl 0,1 N contenant de l'alcool et de préférence une quantité d'éthanol comprise entre 4 et 40% (par exemple 10, 20, 30 ou 40%), n'est pas supérieur de plus de 15 points (15% en valeur absolue) comparé à celui libéré dans un milieu d'acide HCl 0,1 N.

L'invention concerne également une forme pharmaceutique orale à libération prolongée d'au moins un principe actif, comprenant des microgranules selon l'invention.

Plus particulièrement, l'invention porte sur l'utilisation d'une forme pharmaceutique orale selon l'invention pour éviter ou limiter une décharge immédiate du principe actif induite par la consommation d'alcool lors de l'administration de cette forme pharmaceutique.

L'invention porte également sur une forme pharmaceutique de l'invention pour son utilisation en tant que médicament administré par voie orale en prise unique une fois par jour.

Un autre objet de l'invention concerne un procédé de préparation des microgranules de l'invention.

Dans le cadre de la présente invention, le support neutre peut être soluble dans l'eau ou dans une solution alcoolique mais il peut également être insoluble dans l'eau ou dans une solution alcoolique ou être rendu insoluble dans l'eau ou dans une solution alcoolique grâce à une couche de pré-montage.

Des supports neutres insolubles dans l'eau ou dans une solution alcoolique appropriés comprenant des supports à base d'au moins un excipient de nature hydrophobe choisi parmi : la cellulose, les dérivés de la cellulose (cellulose microcristalline), les dérivés des phosphates (phosphates de calcium), la silice et les dérivés des silicates (silicate de magnésium, silicate d'aluminium et leurs mélanges), la cire de Carnauba, le polyvinyl alcool ou tout autre support insoluble.

Des supports neutres solubles dans l'eau ou dans une solution alcoolique appropriés comprennent des supports à base d'au moins un excipient choisi parmi : l'amidon, le saccharose, les polyols tels que le mannitol ou le lactose et leurs mélanges.

Le support neutre peut également être rendu insoluble dans l'eau ou dans une solution alcoolique en recouvrant un neutre d'une couche de pré-montage comprenant au moins un polymère hydrophobe, au moins une charge inerte et éventuellement un agent tensio-actif et/ou un agent plastifiant.

Le ou les principes actifs sont intégrés dans la couche active en association avec un agent liant pharmaceutiquement acceptable, tel que ceux habituellement utilisés dans l'industrie pharmaceutique pour la fixation de principes actifs à la surface de supports neutres. Ainsi, la méthode de fixation de la couche active décrite dans le brevet EP 1 200 071 peut tout à fait être employée pour la fixation de la couche active dans le cadre de la présente invention.

De façon préférée, la couche active des microgranules conformes à l'invention est appliquée par pulvérisation d'une dispersion de principe actif dans un solvant (appelée dispersion de montage).

Parmi les agents liants pharmaceutiquement acceptables, on utilisera préférentiellement des agents liants de nature hydrophile et notamment des dérivés de la cellulose tels que l'HPMC, en particulier les grades Pharmacoat® 603 et Pharmacoat® 606, ou l'hydroxypropylcellulose ou l'hydroxyéthylcellulose, des dérivés de la polyvinylpyrrolidone, en particulier le grade PVP K 30 et également des dérivés du polyéthylene glycol, en particulier le polyéthylene glycol dont le poids moléculaire vaut entre 600 et 7000, tels que le PEG4000 et le PEG6000 notamment, et leurs mélanges, des dérivés vinyliques tels que le polyvinylalcool.

Le solvant de la dispersion de montage pulvérisée doit être adapté au principe actif ou au mélange de principes actifs employés. Ainsi, on pourra par exemple utiliser l'eau, des solvants organiques, parmi eux l'éthanol ou des solutions hydro- alcooliques de diverses concentrations pour la réalisation de la solution à la base de la couche active.

De préférence, le pourcentage massique de liant dans la couche active par rapport au principe actif est compris entre 25 % et 200 % m/m, de préférence entre 50 % et 100 % m/m.

Un agent tensioactif peut être ajouté à la phase de montage pour améliorer la solubilité du principe actif ou stabiliser la suspension de montage.

L'agent tensioactif est utilisé en quantités de 0 à 50% et préférentiellement entre 0 et 20%. Parmi les tensioactifs utilisables, peuvent être cités les sels alcalins ou alcalinoterreux des acides gras, le sodium dodécyl sulfate et le docusate de sodium étant préférés, les huiles polyoxyéthylénées, de préférence l'huile de ricin hydrogénée polyoxyéthylénée, les copolymères polyoxyéthylène- polyoxypropylène, les esters de sorbitan polyoxyéthylénés, les dérivés de l'huile de ricin polyoxyéthylénés, les stéarates, de préférence de calcium, de magnésium, d'aluminium ou de zinc, les polysorbates, les stéarylfumarates, de préférence de sodium, le béhénate de glycérol, le chlorure de benzalkonium, le bromure d'acétyltriméthyl ammonium, l'alcool cétylique et leurs mélanges.

Dans la mesure du possible, il est préférable d'utiliser des solvants non toxiques et facilement éliminables par évaporation lors du séchage afin qu'il n'en subsiste aucune trace dans les microgranules.

L'enrobage permettant de contrôler la libération contient un polymère hydrophobe empêchant la libération immédiate du principe actif en quantité comprise préférentiellement entre 30% à 80%, de manière encore préférée de 50% à 80%, de la masse sèche de ladite couche d'enrobage.

Le taux d'enrobage représente le rapport de la quantité de masse sèche constituant l'enrobage assurant une libération prolongée du principe actif sur la masse totale du microgranule avant enrobage (en masse sèche). Le taux d'enrobage est compris entre 0,1 % à 70% m/m, de préférence, de 2% à 50% m/m, et, plus préférentiellement encore, de 10% à 40%. Ou autrement dit, le rapport entre la masse de vernis sec (= polymère et éventuels additifs en masse sèche) constituant l'enrobage empêchant la libération immédiate du principe actif sur la masse totale du microgranule avant enrobage (en masse sèche) est compris entre 0,1 % à 70% m/m, de préférence, de 2% à 50% m/m, et, plus préférentiellement encore, de 10% à 40%.

Les polymères utilisés pour assurer une libération prolongée du principe actif sont des dérivés non hydrosolubles de la cellulose, de préférence, sélectionnés dans le groupe de produits suivants : l'éthylcellulose (Aquacoat ECD30), l'acétate butyrate de cellulose, l'acétate de cellulose et leurs mélanges.

Dans un mode de réalisation particulier de l'invention, l'enrobage à libération prolongé des microgranules ne comprend pas de poly(meth)acrylate , en particulier pH-dépendant, tel que l'Eudragit ® L100-55 (poly(methacrylic acid, ethyl acrylate) 1 :1).

Dans un mode de réalisation particulier de l'invention, le polymère assurant la libération prolongée du principe actif est constitué d'un ou plusieurs dérivés non hydrosoluble(s) de la cellulose.

La couche d'enrobage comprend également au moins 20% d'une charge inerte par rapport à la masse sèche du polymère enrobage.

On entend par « charge inerte » un agent solide à température ambiante, pharmaceutiquement acceptable, non soluble dans l'eau et qui permet de réduire la perméabilité de la forme pharmaceutique à laquelle elle est incorporée.

De préférence, la charge inerte est incorporée à l'enrobage sous forme de poudre, en particulier sous une forme micronisée.

Avantageusement, la charge inerte uniformément répartie dans la couche d'enrobage est choisie dans le groupe comprenant notamment le talc, le stéarate de magnésium, le monostéarate de glycérol, la silice et les dérivés des silicates (silicate de magnésium, silicate d'aluminium), le stéarylfumarate de magnésium et leurs mélanges.

Selon des modes de réalisation particuliers de l'invention, la quantité de charge inerte par rapport à la masse sèche de polymère hydrophobe est supérieure à 50 %, supérieure à 60 %, comprise entre 51 % et 155%, entre 61 % et 150 %, entre 61 et 109%, entre 65 % et 115 %, entre 65 % et 109 %, entre 70 % et 105 %, entre 80 % et 100 %, ou entre 85 % et 95 %.

Selon un mode de réalisation particulier de l'invention, le support neutre est soluble dans l'eau ou dans une solution alcoolique et la quantité de charge inerte par rapport à la masse sèche de polymère hydrophobe est supérieure à 30 % et inférieure ou égale à 155 %, de préférence inférieure à 120%.

L'agent tensioactif est optionnellement présent dans l'enrobage à raison de 0 à 30% m/m, de préférence de 0 à 20% m/m, et, plus préférentiellement encore, de 5 à 15% de la masse sèche de polymère d'enrobage. L'agent tensioactif est de préférence sélectionné dans le groupe de produits suivants : les sels alcalins ou alcalinoterreux des acides gras, le sodium dodécyl sulfate et le docusate de sodium étant préférés, les huiles polyoxyéthylénées, de préférence l'huile de ricin hydrogénée polyoxyéthylénée, les copolymères polyoxyéthylène- polyoxypropylène, les esters de sorbitan polyoxyéthylénés, les dérivés de l'huile de ricin polyoxyéthylénés les stéarates, de préférence de calcium, de magnésium, d'aluminium ou de zinc, les polysorbates, les stéarylfumarates, de préférence de sodium, le béhénate de glycérol, le chlorure de benzalkonium, le bromure d'acétyltriméthyl ammonium, l'alcool cétylique et leurs mélanges.

L'agent plastifiant est également optionnellement présent dans l'enrobage et peut être ajouté à la dispersion d'enrobage à raison de 0% à 50% m/m, de préférence, de 2% à 25% m/m, en masse sèche de polymère d'enrobage.

L'agent plastifiant est sélectionné notamment dans le groupe de produits suivants : le glycérol et ses esters, de préférence dans le sous-groupe suivant : les triglycérides à chaînes moyennes, les glycérides acétylés, glycéryl-mono- stéarate, glycéryl-thacétate, glycéryl -tributyrate, les phtalates, de préférence dans le sous-groupe suivant : dibutylphtalate, diéthylphtalate, diméthylphtalate, dioctylphtalate, les citrates, de préférence dans le sous-groupe suivant : acétyltributylcitrate, acétylthéthylcitrate, tributylcitrate, triéthylcitrate, les sébacates, de préférence dans le sous-groupe suivant : diéthylsébacate, dibutylsébacate, les adipates, les azélates, les benzoates, le chlorobutanoles polyéthylène glycols, les huiles végétales, les fumarates, de préférence le diéthylfumarate, les malates, de préférence le diéthylmalate, les oxalates, de préférence le diéthyloxalate, les succinates ; de préférence le dibutylsuccinate, les butyrates, les esters de l'alcool cétylique, les malonates, de préférence le diéthylmalonate, l'huile de ricin, et leurs mélanges.

Plus préférentiellement, l'agent plastifiant est sélectionné dans le groupe de produits suivants : les monoglycérides acétylés notamment le Myvacet(R) 9-45, le triéthyl citrate (TEC), le dibutylsébacate, la triacétine et leurs mélanges.

### Le principe actif

La couche active constituant les microgranules conformes à l'invention comprend au moins un principe actif pharmaceutique qui peut être de toute nature.

Les microgranules selon la présente invention peuvent comprendre en tant que principe actif, les hormones ou leurs dérivés par exemple, les principes actifs agissant sur le système nerveux central, les principes actifs agissant sur le système cardiovasculaire, les antibiotiques, les anti-viraux et les analgésiques.

Les principes actifs agissant sur le système nerveux central sont de préférence choisis parmi les anti-épileptiques, les anti-parkinsoniens, les psycho-stimulants, les psychotropes, les antidépresseurs, les anxiolytiques et les anti-psychotiques par exemple.

Les principes actifs agissant sur le système cardiovasculaire sont de préférence choisis parmi les anti-hypertenseurs, les anti-thrombotiques, les anti-agrégants et les hypocholestérolémiants notamment.

Les antibiotiques peuvent être choisis parmi les bêta-latamines, les cyclines, les aminosides, les macrolides, les quinolones, les antibiotiques glycopeptidiques, les imidazolés, les sulfamides, les antituberculeux et les antilépreux notamment.

Les antiviraux peuvent être choisis parmi les inhibiteurs de la réplication ou de la multiplication virale notamment.

Les analgésiques peuvent être choisis parmi les analgésiques non opiacés, opiacés faibles, opioïdes mixtes, morphiniques ou spasmodiques, notamment l'hydrocodone, l'hydromorphone, la morphine, l'oxycodone, l'oxymorphone, le tramadol, la gabapentine et leurs dérivés.

Dans un mode de réalisation particulier de l'invention, les microgranules ne comprennent pas d'agent aversif, tel que le chlorhydrate de rimonabant. On entend par agent aversif, un agent qui provoque une réaction physiologique ou psychologique désagréable, notamment en association avec de l'alcool ou bien avec une substance psychoactive comme le cannabis.

Dans un autre mode de réalisation particulier de l'invention, les microgranules ne comprennent pas de metformine ou d'acyclovir.

### Le procédé de préparation des microgranules

La présente invention a en outre pour objet le procédé de préparation des microgranules précédemment décrits qui comprend les étapes suivantes :
- l'introduction de supports sphériques neutres solubles, insolubles ou rendus insolubles dans une enceinte à lit fluidisé, par exemple,
- la pulvérisation sur ces supports sphériques neutres d'au moins un principe actif en solution ou en suspension dans un solvant organique et/ou aqueux additionné d'au moins un polymère hydrosoluble ou non hydrosoluble (agent liant),
- la pulvérisation d'une suspension d'enrobage comprenant au moins un polymère hydrophobe et une charge inerte sur les particules enrobées obtenues à l'étape précédente,
- éventuellement, le séchage des microgranules médicamenteux ainsi obtenus.

### Préparation de la dispersion de montage

L'étape dite de montage de la couche active conformément à la présente invention permet d'obtenir des microgranules dont la teneur en actif est à la fois précise et uniforme.

La dispersion dite de montage est la dispersion dans laquelle le ou les principes actifs vont être dissous ou mis en suspension (dispersés) et qui va être pulvérisée à la surface des microgranules. Cette dispersion contient également un agent liant conventionnel dissous.

### Montage de la couche active

Le principe actif est appliqué sur les granules de façon conventionnelle par pulvérisation, en lit fluidisé ou en turbine perforée par exemple. D'une manière générale, ce procédé repose sur la pulvérisation simultanée au travers d'une buse, du ou des principes actifs et éventuellement d'un liant qui sont dissous ou dispersés dans la solution de montage, ce qui garantit pour cette étape du procédé une parfaite homogénéité de teneur. Le temps nécessaire au montage est très variable et dépend de la quantité d'actif à pulvériser et de sa solubilité dans la solution de montage. De manière générale il est compris entre 1 et 48 heures.

A l'issue de l'étape de montage, les microgranules sont séchés en lit fluidisé ou en turbine perforée puis tamisés.

### Enrobage des microgranules

Le polymère d'enrobage est appliqué sur les microgranules précédents de façon conventionnelle par pulvérisation, en lit fluidisé ou en turbine perforée par exemple. D'une manière générale, ce procédé repose sur la pulvérisation simultanée au travers d'une buse, du ou des polymères d'enrobage, d'une charge inerte, optionnellement d'un agent tensioactif et/ou d'un plastifiant qui sont dissous ou dispersés dans un solvant adapté.

Une solution organique de polymère peut être utilisée pour l'enrobage : dans ce cas, l'utilisation d'un plastifiant n'est pas forcément requise.

Si le véhicule est l'eau, une dispersion aqueuse de polymère est utilisée, le procédé consiste alors en la pulvérisation de la dispersion, un séchage dans le même équipement et, si nécessaire, une étape de maturation du film d'enrobage (aussi appelée curing) qui permet l'obtention d'un film homogène et uniforme. Le curing peut être réalisé en lit fluidisé, en turbine perforée ou en étuve par exemple.

Le temps nécessaire à l'enrobage est très variable et dépend de la quantité de polymère à pulvériser. A l'issue de l'étape d'enrobage, les microgranules sont séchés en lit fluidisé puis tamisés.

### Couche de Pré-montage

Selon un autre aspect de l'invention et comme décrit plus haut, chaque microgranule peut comprendre au moins une couche de pré-montage, située entre le support neutre et la couche de montage, afin de rendre le support neutre insoluble.

Ainsi le support neutre rendu insoluble est obtenu en recouvrant un neutre d'une couche de pré-montage comprenant au moins un polymère hydrophobe, au moins une charge inerte et éventuellement un agent tensio- actif et/ou un agent plastifiant.

Le ou les polymère(s) hydrophobe(s), charge(s) inerte(s), agent(s) plastifiant(s), et agent(s) tensio-actif(s) sont identiques à ceux décrits plus haut.

Le polymère hydrophobe présent dans la couche de pré-montage sera compris entre 40% à 100%, de préférence de 50% à 80%, de la masse sèche de la couche de pré-montage.

La charge inerte peut être présente en une quantité supérieure à 50 %, supérieure à 60 %, comprise entre 51 % et 155%, entre 61 % et 150 %, entre 61 et 109%, entre 65 % et 115 %, entre 65 % et 109 %, entre 70 % et 105 %, entre 80 % et 100 %, ou entre 85 % et 95 % de la masse sèche de polymère hydrophobe.

Si le support neutre est soluble dans l'eau ou dans une solution alcoolique, la quantité de charge inerte par rapport à la masse sèche de polymère hydrophobe peut être supérieure à 30 % et inférieure ou égale à 155 %, de préférence inférieure à 120 %.

L'agent plastifiant contenu dans la couche de pré-montage peut être ajouté à raison de 0% à 50% m/m, de préférence, de 2% à 25% m/m, en masse sèche de polymère hydrophobe.

Un agent tensio-actif peut également être ajouté à la couche de pré-montage à raison 0 à 30% m/m, de préférence de 0 à 20% m/m, et, plus préférentiellement encore, de 5 à 15% de la masse sèche de polymère hydrophobe.

Le polymère hydrophobe est appliqué sur les neutres de façon conventionnelle par pulvérisation, en lit fluidisé ou en turbine perforée par exemple. D'une manière générale, ce procédé repose sur la pulvérisation simultanée au travers d'une buse, du ou des polymères hydrophobes, d'une charge inerte, optionnellement d'un plastifiant et/ou d'un agent tensioactif qui sont dissous ou dispersés dans un solvant adapté.

Une solution organique de polymère peut être utilisée pour appliquer la couche de pré-montage, dans ce cas, l'utilisation d'un plastifiant n'est pas forcément requise.

Si le véhicule est l'eau, une dispersion aqueuse de polymère est utilisée, le procédé consiste alors en la pulvérisation de la dispersion, un séchage dans le même équipement et, si nécessaire, une étape de maturation du film d'enrobage (aussi appelée curing) qui permet l'obtention d'un film homogène et uniforme. Le curing peut être réalisé en lit fluidisé, en turbine perforée ou en étuve par exemple.

### Les tests de dissolution et de dosage

D'une manière générale, les conditions de dosage et de dissolution des microgranules conformes à l'invention sont celles prescrites par les différentes pharmacopées, notamment européenne, américaine ou japonaise.

Ainsi, pour déterminer les cinétiques de libération des différents systèmes étudiés, un appareil de dissolution thermostaté conventionnel à palettes ou à paniers peut être employé. Les unités médicamenteuses sont introduites dans chaque vase et des prélèvements sont effectués périodiquement pour déterminer la quantité de principe actif libéré au cours du temps. Les prélèvements peuvent être manuels ou automatiques et les analyses peuvent être réalisées directement avec un spectrophotomètre UV/visible ou après séparation en CLHP (chromatographie liquide haute performance) couplée à une détection UV/visible par exemple.

### BREVE DESCRIPTION DES FIGURES

Figure 1 : Profils de dissolution de microgranules contenant du Sulfate de Morphine et enrobés d'éthylcellulose, de triéthylcitrate et de talc dans différents milieux (taux d'enrobage 15%, taux de charge inerte de 50%).
Figure 2 : Profils de dissolution de microgranules contenant du sulfate de Morphine et enrobés d'éthylcellulose, de triéthylcitrate et de talc dans différents milieux (taux d'enrobage 20%, taux de charge inerte de 50%).
Figure 3 : Profils de dissolution de microgranules contenant du sulfate de Morphine et enrobés d'éthylcellulose, de triéthylcitrate et de talc dans différents milieux (taux d'enrobage 20%, taux de charge inerte de 25%).
Figure 4 : Profils de dissolution de microgranules contenant du sulfate de Morphine et enrobés d'éthylcellulose, de triéthylcitrate et de talc (taux d'enrobage 15%, taux de charge inerte de 50%) dans différents milieux, les microgranules ne comprenant pas de couche de pré-montage.
Figure 5 : Profils de dissolution de microgranules contenant du sulfate de Morphine et enrobés d'éthylcellulose, de triéthylcitrate et de talc (taux d'enrobage 15%, taux de charge inerte de 90%) dans différents milieux, les microgranules ne comprenant pas de couche de pré-montage.
Figure 6 : Profils de dissolution de microgranules contenant du sulfate de Morphine et enrobés d'éthylcellulose, de triéthylcitrate et de talc (taux d'enrobage 15%, taux de charge inerte de 120%) dans différents milieux, les microgranules ne comprenant pas de couche de pré-montage.
Figure 7 : Profils de dissolution de microgranules contenant du sulfate de Morphine et enrobés d'éthylcellulose, de triéthylcitrate et de talc (taux d'enrobage 15%, taux de charge inerte de 150%) dans différents milieux, les microgranules ne comprenant pas de couche de pré-montage.
Figure 8 : Profils de dissolution de microgranules comprenant du sulfate de Morphine et enrobés d'éthylcellulose et de triéthylcitrate (taux d'enrobage 10%) dans différents milieux, la couche d'enrobage ne contenant pas de talc.
Figure 9 : Profils de dissolution de microgranules comprenant du sulfate de Morphine et enrobés d'éthylcellulose et de triéthylcitrate (taux d'enrobage 15%) dans différents milieux, la couche d'enrobage ne contenant pas de talc.
Figure 10 : Profils de dissolution de microgranules de Sulfate de Morphine et enrobés d'éthylcellulose et de triéthylcitrate (taux d'enrobage 15%) dans différents milieux, les microgranules ne comprenant pas de couche de pré-montage et contenant du talc en dehors de la couche d'enrobage.

### EXEMPLES

### Exemple 1 : Microgranules de sulfate de Morphine à libération prolongée résistants à l'alcool (Taux d'enrobage 15%, exprimé en masse sèche de polymère d'enrobage par rapport à la masse de microgranules avant enrobage, taux de charge inerte de 50% par rapport à la masse sèche du polymère d'enrobage hydrophobe)

### a) Préparation des microgranules de Sulfate de Morphine à libération prolongée

Le principe actif utilisé est le sulfate de Morphine. Les noyaux neutres utilisés sont des sphères de sucre (Neutres SP NPPHARM). La taille de ces supports est de l'ordre de 400 à 500 µm. L'agent liant utilisé est l'hydroxypropylméthylcellulose (HPMC 603). Elle est solubilisée dans l'eau puis le sulfate de Morphine est ajouté à cette solution aqueuse, constituant la solution de montage. La solution de montage est pulvérisée en lit fluidisé (Glatt), de même que la suspension d'enrobage et de pré-montage.

Les compositions qualitative et quantitative des microgranules de Sulfate de Morphine sont récapitulées dans le tableau suivant.

| Microgranules (taux d'enrobage 15%) | | Quantités (g) |
|---|---|---|
| PRE-MONTAGE | Neutres SP | 223,53 |
| | éthylcellulose | 170,97 |
| | *éq. masse sèche* | *51,29* |
| | Triéthylcitrate | 12,88 |
| | Talc Pharma | 25,56 |
| MONTAGE | Sulfate de | 313,30 |
| | Morphine HPMC 603 | 109,64 |
| ENROBAGE | éthylcellulose | 367,43 |
| | *éq. masse sèche* | *110,23* |
| | Triéthylcitrate | 26,48 |
| | Talc Pharma | 54,95 |

| Bilan global | % |
|---|---|
| Neutres SP | 24,1 |
| Sulfate de Morphine | 33,8 |
| HPMC 603 | 11,8 |
| éthylcellulose | 17,4 |
| Triéthylcitrate | 4,2 |
| Talc Pharma | 8,7 |

### b) Dosage et dissolution des microgranules

Les essais de libération in vitro du principe actif sont réalisés dans un appareil de dissolution à pales tournantes (Pharmacopée Européenne, Sotax AT7, logiciel IDIS). L'analyse se fait avec un spectrophotomètre UV / visible à une longueur d'onde de 285 et 310 nm (Kontron Instruments spectrophotometer, UVIKON 922). Les échantillons sont soumis à une agitation constante dans des vases contenant chacun 500mL de milieu de dissolution, et la température est maintenue constante à 37°C (+/-0,5°C). Les milieux de dissolution utilisés sont composés soit d'HCl 0,1 N, soit d'un mélange HCl 0,1 N / Ethanol absolu avec une concentration en éthanol absolu égale à 10, 20 ou 40% (v/v). La vitesse de rotation des pales est fixée à 100 tours/min. Des prélèvements sont réalisés en continu sur 24h dans chacun des vases de l'appareil. Pour chaque vase, la prise d'essai de microgranules est équivalente à 120mg de PA.

### c) Profils obtenus dans HCl 0.1 N et les mélanges HCl 0, N / Ethanol absolu concentrés à 10, 20 et 40 % (v/v)

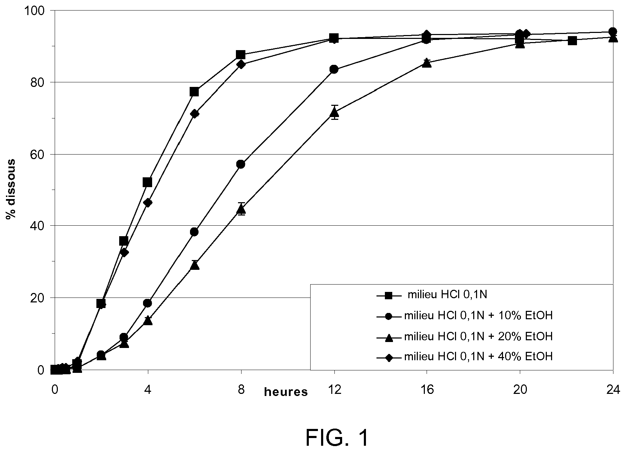

Les profils de dissolution obtenus dans l'HCl 0,1 N et les mélanges HCl 0,1 N / Ethanol absolu concentrés à 10, 20 et 40 % (v/v) en éthanol absolu pour les microgranules présentant un taux d'enrobage de 15% sont consignés sur la figure 1.

La figure 1 montre que les microgranules présentent en effet une libération prolongée dans les milieux ayant une concentration en éthanol de 0, 10, 20 et 40% (v/v).

L'écart des pourcentages de Sulfate de Morphine libérée dans les milieux acido-alcooliques ou dans l'HCl 0,1 N pour le temps 2h sont inférieurs à 15% pour les trois concentrations en éthanol ce qui démontre que ces microgranules sont résistants à l'alcool.

### Exemple 2 : Microgranules de Sulfate de Morphine à libération prolongée résistants à l'alcool (taux d'enrobage de 20%, exprimé en masse sèche de polymère d'enrobage par rapport à la masse de microgranules avant enrobage, taux de charge inerte de 50% par rapport à la masse sèche du polymère d'enrobage hydrophobe)

Selon une variante de l'exemple 1, les microgranules de Sulfate de Morphine résistants à l'alcool peuvent être obtenus en réalisant un taux d'enrobage de 20%. Les méthodes de préparation, de dosage et de dissolution des microgranules restent identiques à l'exemple 1, à l'exception de leur composition quantitative récapitulée dans le tableau suivant.

| Microgranules (taux d'enrobage 20%) | | Quantités (g) |
|---|---|---|
| PRE-MONTAGE | Neutres SP | 223,53 |
| | éthylcellulose | 170,97 |
| | *éq. masse sèche* | *51,29* |
| | Triéthylcitrate | 12,88 |
| | Talc Pharma | 25,56 |
| MONTAGE | Sulfate de | 313,30 |
| | Morphine HPMC 603 | 109,64 |
| ENROBAGE | éthylcellulose | 489,9 |
| | *éq. masse sèche* | 146,97 |
| | Triéthylcitrate | 35,3 |
| | Talc Pharma | 73,6 |

| Bilan global | % |
|---|---|
| Neutres SP | 22,5 |
| Sulfate de Morphine | 31,6 |
| HPMC 603 | 11,0 |
| éthylcellulose | 20,0 |
| Triéthylcitrate | 4,9 |
| Talc Pharma | 10,0 |

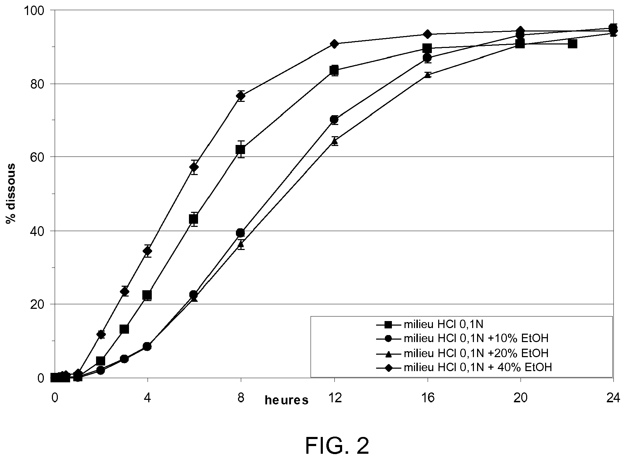

Les profils de dissolution obtenus dans l'HCl 0,1 N et le mélange HCl 0,1 N / Ethanol absolu concentré à 10, 20 et 40 % (v/v) en éthanol absolu pour les microgranules présentant un taux d'enrobage de 20% sont consignés sur la figure 2. L'écart maximum des pourcentages d'actif libéré au bout de 2 heures dans l'HCl 0,1 N par rapport aux milieux acido-alcooliques est de 7,1%, soit inférieur à 15% ce qui démontre que ces microgranules sont résistants à l'alcool.

### Exemple 3 : Microgranules de Sulfate de Morphine à libération prolongée résistants à l'alcool (taux d'enrobage de 20%, exprimé en masse sèche de polymère d'enrobage par rapport à la masse de microgranules avant enrobage, taux de charge inerte de 25% par rapport à la masse sèche du polymère d'enrobage hydrophobe)

Selon une variante des exemples 1 et 2, les microgranules de Sulfate de Morphine résistants à l'alcool sont obtenus en diminuant la quantité de charge inerte dans l'enrobage. Les méthodes de préparation, de dosage et de dissolution des microgranules restent identiques à l'exemple 1, à l'exception de leur composition quantitative récapitulée dans le tableau suivant.

| Microgranules (taux d'enrobage 20%) | | Quantités (g) |
|---|---|---|
| PRE-MONTAGE | Neutres SP | 242,90 |
| | éthylcellulose | 185,80 |
| | *éq. masse sèche* | 55,74 |
| | Triéthylcitrate | 13,99 |
| | Talc Pharma | 27,77 |
| MONTAGE | Sulfate de | 340,45 |
| | Morphine HPMC 603 | 119,14 |
| ENROBAGE | éthylcellulose | 535,87 |
| | *éq. masse sèche* | *160, 76* |
| | Triéthylcitrate | 38,40 |
| | Talc Pharma | 40,13 |

| Bilan global | % |
|---|---|
| Neutres SP | 23,4 |
| Sulfate de Morphine | 32,8 |
| HPMC 603 | 11,5 |
| éthylcellulose | 20,8 |
| Triéthylcitrate | 5,0 |
| Talc Pharma | 6,5 |

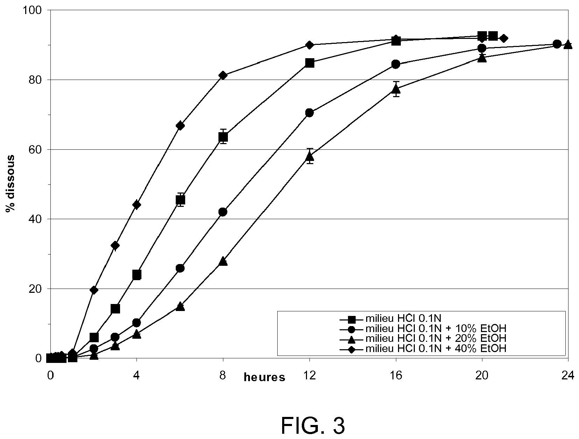

Les profils de dissolution obtenus dans l'HCl 0,1 N et le mélange HCl 0,1 N / Ethanol absolu concentré à 10, 20 et 40 % (v/v) en éthanol absolu pour les microgranules présentant un taux d'enrobage de 20% sont consignés sur la figure 3. L'écart maximum des pourcentages d'actif libéré au bout de 2 heures dans l'HCl 0,1 N par rapport aux milieux acido-alcooliques est de 13,5%, ce qui démontre que ces microgranules sont résistants à l'alcool.

### Exemple 4 : Microgranules de Sulfate de Morphine à libération prolongée résistants à l'alcool (taux d'enrobage de 15%, exprimé en masse sèche de polymère d'enrobage par rapport à la masse de microgranules avant enrobage, taux de charge inerte de 50% par rapport à la masse sèche du polymère d'enrobage hydrophobe) dépourvu de couche de pré-montage.

Selon une variante des exemples 1 et 2, les microgranules de Sulfate de Morphine résistants à l'alcool sont dépourvus de couche de pré-montage. Les méthodes de préparation, de dosage et de dissolution des microgranules restent identiques à l'exemple 1, à l'exception de la taille des neutres de sucres utilisés (Suglets #30, NPPHARM, taille 400 à 600µm) et de leur composition quantitative récapitulée dans le tableau suivant.

| Microgranules (taux d'enrobage 15%) | | Quantités (g) |
|---|---|---|
| MONTAGE | Neutres # 30 | 479,89 |
| | Sulfate de Morphine | 236,98 |
| | HPMC 603 | 83,13 |
| ENROBAGE | éthylcellulose | 400,00 |
| | *éq. masse sèche* | *120,00* |
| | Triéthylcitrate | 28,80 |
| | Talc Pharma | 60,00 |

| Bilan global | % |
|---|---|
| Neutres # 30 | 47,6 |
| Sulfate de Morphine | 23,5 |
| HPMC 603 | 8,2 |
| éthylcellulose | 11,9 |
| Triéthylcitrate | 2,9 |
| Talc Pharma | 5,9 |

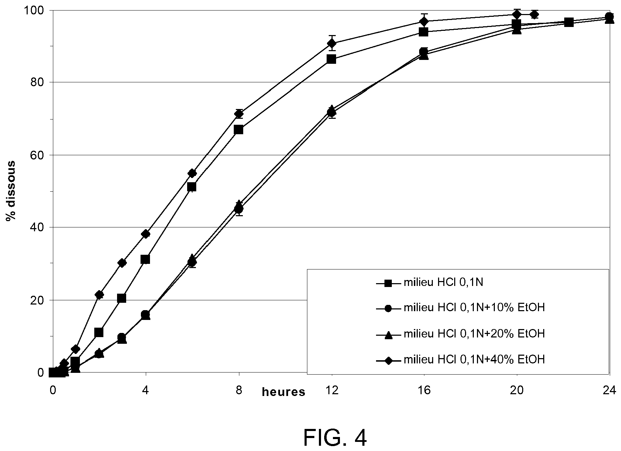

Les profils de dissolution obtenus dans l'HCl 0,1 N et le mélange HCl 0,1 N / Ethanol absolu concentré à 10, 20 et 40 % (v/v) en éthanol absolu pour les microgranules sans couche de pré-montage et présentant un taux d'enrobage de 15% sont consignés sur la figure 4. L'écart des pourcentages d'actif libéré au bout de 2 heures dans l'HCl 0,1 N par rapport aux milieux acido-alcoolique est de 10,5 %, soit inférieur à 15%, ce qui démontre que ces microgranules sont résistants à l'alcool.

La figure 4 montre que les microgranules dépourvus de couche de pré-montage présentent également une libération prolongée dans les milieux ayant une concentration en éthanol de 0, 10, 20 et 40% (v/v).

### Exemple 5 : Microgranules de Sulfate de Morphine à libération prolongée résistants à l'alcool (taux d'enrobage de 15%, exprimé en masse sèche de polymère d'enrobage par rapport à la masse de microgranules avant enrobage, taux de charge inerte de 90% par rapport à la masse sèche du polymère d'enrobage hydrophobe) dépourvu de couche de pré-montage.

Selon une variante de l'exemple 4, les microgranules de Sulfate de Morphine résistants à l'alcool sont obtenus en augmentant la quantité de charge inerte dans l'enrobage. Les méthodes de préparation, de dosage et de dissolution des microgranules restent identiques à l'exemple 1, à l'exception de la taille des neutres de sucres utilisés (Suglets #30, NPPHARM, taille 400 à 600µm) et de leur composition quantitative récapitulée dans le tableau suivant.

| Microgranules (taux d'enrobage 15%) | | Quantités (g) |
|---|---|---|
| MONTAGE | Neutres # 30 | 479,89 |
| | Sulfate de Morphine | 236,98 |
| | HPMC 603 | 83,13 |
| ENROBAGE | Ethylcellulose | 400,00 |
| | *éq. masse sèche* | *120,00* |
| | Triéthylcitrate | 29,00 |
| | Talc Pharma | 108,00 |

| Bilan global | % |
|---|---|
| Neutres # 30 | 45,4 |
| Sulfate de Morphine | 22,4 |
| HPMC 603 | 7,9 |
| éthylcellulose | 11,4 |
| Triéthylcitrate | 2,7 |
| Talc Pharma | 10,2 |

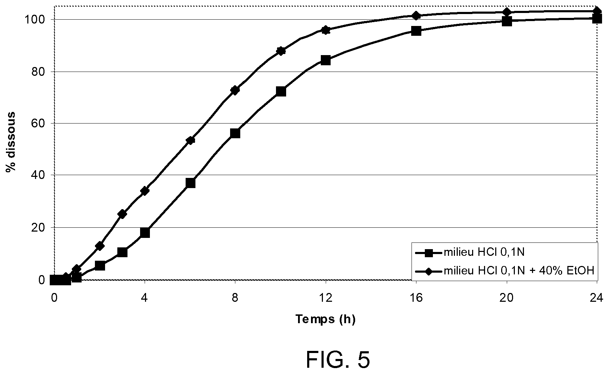

Les profils de dissolution obtenus dans l'HCl 0,1 N et le mélange HCl 0,1 N / Ethanol absolu concentré à 40 % (v/v) en éthanol absolu pour les microgranules sans couche de pré-montage, présentant un taux d'enrobage de 15% et un taux de charge de 90% sont consignés sur la figure 5. L'écart des pourcentages d'actif libéré au bout de 2 heures dans l'HCl 0,1 N par rapport aux milieux acido-alcoolique est de 7,7 %, soit inférieur à 15%, ce qui démontre que ces microgranules sont résistants à l'alcool.

### Exemple 6 : Microgranules de Sulfate de Morphine à libération prolongée résistants à l'alcool (taux d'enrobage de 15%, exprimé en masse sèche de polymère d'enrobage par rapport à la masse de microgranules avant enrobage, taux de charge inerte de 120% par rapport à la masse sèche du polymère d'enrobage hydrophobe) dépourvu de couche de pré-montage.

Selon une variante des exemples 4 et 5, les microgranules de Sulfate de Morphine résistants à l'alcool sont obtenus en augmentant la quantité de charge inerte dans l'enrobage. Les méthodes de préparation, de dosage et de dissolution des microgranules restent identiques à l'exemple 1, à l'exception de la taille des neutres de sucres utilisés (Suglets #30, NPPHARM, taille 400 à 600µm) et de leur composition quantitative récapitulée dans le tableau suivant.

| Microgranules (taux d'enrobage 15%) | | Quantités (g) |
|---|---|---|
| MONTAGE | Neutres # 30 | 479,89 |
| | Sulfate de Morphine | 236,98 |
| | HPMC 603 | 83,13 |
| ENROBAGE | éthylcellulose | 400,00 |
| | *éq. masse sèche* | *120,00* |
| | Triéthylcitrate | 29,00 |
| | Talc Pharma | 144,00 |

| Bilan global | % |
|---|---|
| Neutres # 30 | 43,9 |
| Sulfate de Morphine | 21,7 |
| HPMC 603 | 7,6 |
| éthylcellulose | 11,0 |
| Triéthylcitrate | 2,7 |
| Talc Pharma | 13,2 |

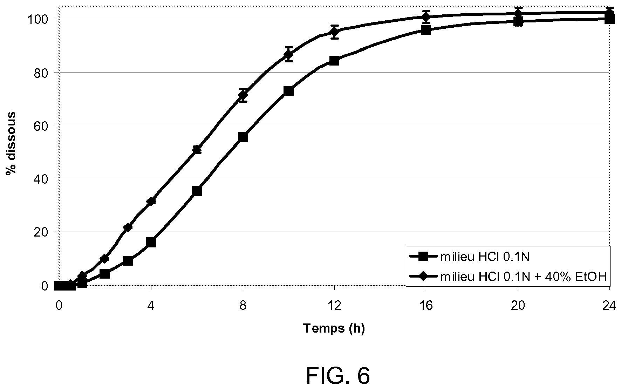

Les profils de dissolution obtenus dans l'HCl 0,1 N et le mélange HCl 0,1 N / Ethanol absolu concentré à 40 % (v/v) en éthanol absolu pour les microgranules sans couche de pré-montage, présentant un taux d'enrobage de 15% et un taux de charge de 120% sont consignés sur la figure 6. L'écart des pourcentages d'actif libéré au bout de 2 heures dans l'HCl 0,1 N par rapport aux milieux acido-alcoolique est de 5,3 %, soit inférieur à 15%, ce qui démontre que ces microgranules sont résistants à l'alcool.

### Exemple 7 : Microgranules de Sulfate de Morphine à libération prolongée résistants à l'alcool (taux d'enrobage de 15%, exprimé en masse sèche de polymère d'enrobage par rapport à la masse de microgranules avant enrobage, taux de charge inerte de 150% par rapport à la masse sèche du polymère d'enrobage hydrophobe) dépourvu de couche de pré-montage.

Selon une variante des exemples 4 à 6, les microgranules de Sulfate de Morphine résistants à l'alcool sont obtenus en augmentant la quantité de charge inerte dans l'enrobage. Les méthodes de préparation, de dosage et de dissolution des microgranules restent identiques à l'exemple 1, à l'exception de la taille des neutres de sucres utilisés (Suglets #30, NPPHARM, taille 400 à 600µm) et de leur composition quantitative récapitulée dans le tableau suivant.

| Microgranules (taux d'enrobage 15%) | | Quantités (g) |
|---|---|---|
| MONTAGE | Neutres # 30 | 479,89 |
| | Sulfate de Morphine | 236,98 |
| | HPMC 603 | 83,13 |
| ENROBAGE | éthylcellulose | 400,00 |
| | *éq. masse sèche* | *120,00* |
| | Triéthylcitrate | 29,00 |
| | Talc Pharma | 180,00 |

| Bilan global | % |
|---|---|
| Neutres # 30 | 42,5 |
| Sulfate de Morphine | 21,0 |
| HPMC 603 | 7,4 |
| éthylcellulose | 10,6 |
| Triéthylcitrate | 2,6 |
| Talc Pharma | 15,9 |

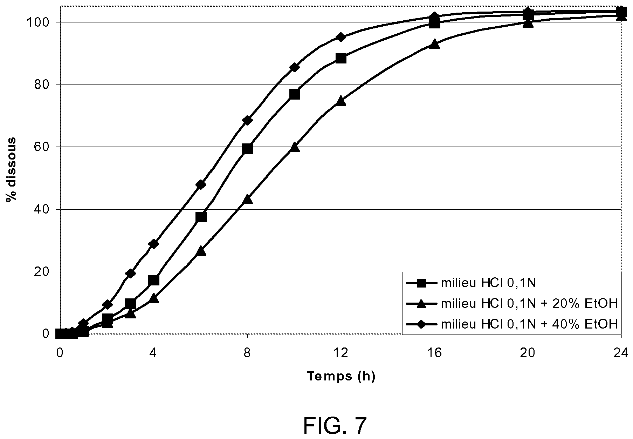

Les profils de dissolution obtenus dans l'HCl 0,1 N et le mélange HCl 0,1 N / Ethanol absolu concentré à 40 % (v/v) en éthanol absolu pour les microgranules sans couche de pré-montage, présentant un taux d'enrobage de 15% et un taux de charge de 150% sont consignés sur la figure 7. L'écart des pourcentages d'actif libéré au bout de 2 heures dans l'HCl 0,1 N par rapport aux milieux acido-alcoolique est de 4,7 %, soit inférieur à 15%, ce qui démontre que ces microgranules sont résistants à l'alcool.

### Contre-exemple 8 : Microgranules de sulfate de Morphine à libération prolongée (taux d'enrobage de 10%, exprimé en masse sèche de polymère d'enrobage par rapport à la masse de microgranules avant enrobage) ne contenant pas de talc dans la couche d'enrobage.

Selon une variante, les microgranules de sulfate de Morphine ne contiennent pas de talc dans la couche d'enrobage.

Les méthodes de préparation, de dosage et de dissolution des microgranules restent identiques à l'exemple 1, à l'exception de leur composition quantitative récapitulée dans le tableau suivant.

| Microgranules (taux d'enrobage 10%) | | Quantités (g) |
|---|---|---|
| PRE-MONTAGE | Neutres SP | 243,15 |
| | Ethylcellulose | 185,80 |
| | *éq. masse sèche* | *55,59* |
| | Triéthylcitrate | 13,89 |
| | Talc Pharma | 27,78 |
| MONTAGE | Morphine sulfate | 340,43 |
| | HPMC 603 | 119,15 |
| ENROBAGE | Ehylcellulose | 266,80 |
| | *éq. masse sèche* | *80,04* |
| | Triéthylcitrate | 19,20 |
| | Talc Pharma | 0,00 |

| Bilan global | % |
|---|---|
| Neutres SP | 27,04 |
| Morphine sulfate | 37,86 |
| HPMC 603 | 13,25 |
| Ethylcellulose | 15,08 |
| Triéthylcitrate | 3,68 |
| Talc Pharma | 3,09 |

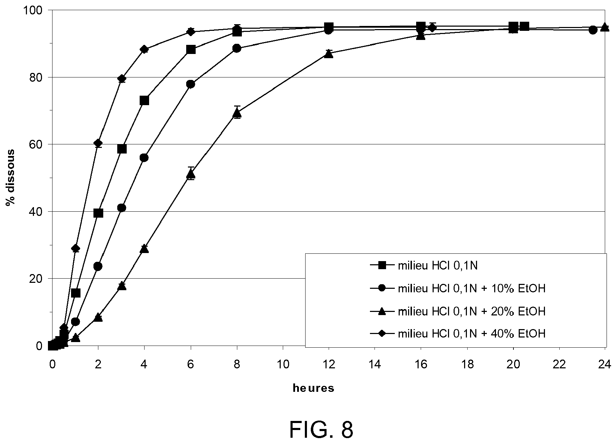

Les profils de dissolution obtenus dans l'HCl 0,1 N et le mélange HCl 0,1 N / Ethanol absolu concentré à 10, 20 et 40 % (v/v) en éthanol absolu pour les microgranules ne contenant pas de talc dans la couche d'enrobage et présentant un taux d'enrobage de 10% sont consignés sur la figure 8. L'écart des pourcentages d'actif libéré au bout de 2 heures dans l'HCl 0,1 N par rapport au milieu HCl 0.1N additionné de 40% d'éthanol est de 20,9 %, soit supérieur à 15%. De plus, le profil de libération obtenu dans le milieu exempt d'alcool n'est pas acceptable au regard d'une prise journalière unique par le patient.

### Contre-exemple 9 : Microgranules de Sulfate de Morphine à libération prolongée résistants à l'alcool (taux d'enrobage de 15%, exprimé en masse sèche de polymère d'enrobage par rapport à la masse de microgranules avant enrobage) ne contenant pas de talc dans la couche d'enrobage.

Selon une variante, les microgranules de Sulfate de Morphine résistants à l'alcool ne contiennent pas de talc dans la couche d'enrobage.

Les méthodes de préparation, de dosage et de dissolution des microgranules restent identiques à l'exemple 1, à l'exception de leur composition quantitative récapitulée dans le tableau suivant.

| Microgranules (taux d'enrobage 15%) | | Quantités (g) |
|---|---|---|
| PRE-MONTAGE | Neutres SP | 243,15 |
| | éthylcellulose | 185,80 |
| | *éq. masse sèche* | *55,59* |
| | Triéthylcitrate | 13,89 |
| | Talc Pharma | 27,78 |
| MONTAGE | Sulfate de Morphine | 340,43 |
| | | 119,15 |
| | HPMC 603 | |
| ENROBAGE | éthylcellulose | 400,60 |
| | *éq. masse sèche* | *120,18* |
| | Triéthylcitrate | 28,90 |
| | Talc Pharma | 0,00 |

| Bilan global | % |
|---|---|
| Neutres SP | 25,6 |
| Sulfate de Morphine | 35,9 |
| HPMC 603 | 12,6 |
| éthylcellulose | 18,5 |
| Triéthylcitrate | 4,5 |
| Talc Pharma | 2,9 |

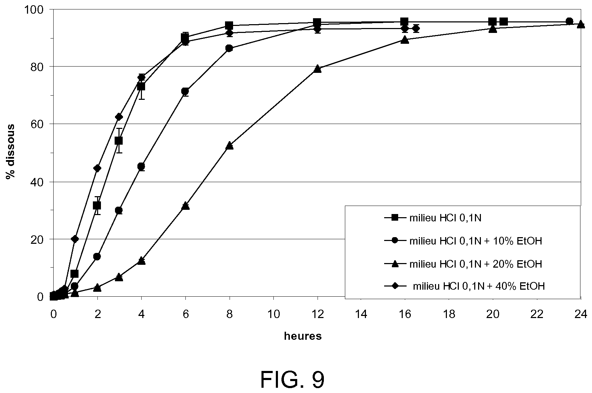

Les profils de dissolution obtenus dans l'HCl 0,1 N et le mélange HCl 0,1 N / Ethanol absolu concentré à 10, 20 et 40 % (v/v) en éthanol absolu pour les microgranules ne contenant pas de talc dans la couche d'enrobage et présentant un taux d'enrobage de 15% sont consignés sur la figure 9. L'écart maximum des pourcentages d'actif libéré au bout de 2 heures dans l'HCl 0,1 N par rapport aux milieux acido-alcooliques est de 13,0 %, soit inférieur à 15%, ce qui démontre que ces microgranules sont résistants à l'alcool. En revanche, le profil de libération obtenu dans le milieu exempt d'alcool n'est pas acceptable au regard d'une prise journalière unique par le patient, même en augmentant le taux d'enrobage de 10 à 15%.

### Contre-exemple 10 : Microgranules de Sulfate de Morphine à libération prolongée (taux d'enrobage de 15%, exprimé en masse sèche de polymère d'enrobage par rapport à la masse de microgranules avant enrobage) contenant du talc en dehors de la couche d'enrobage.

Selon une variante des exemples 4 à 7, les microgranules de Sulfate de Morphine contiennent du talc en dehors de la couche d'enrobage. Les méthodes de préparation, de dosage et de dissolution des microgranules restent identiques à l'exemple 1, à l'exception de la taille des neutres de sucres utilisés (Suglets #30, NPPHARM, taille 400 à 600µm) et de leur composition quantitative récapitulée dans le tableau suivant.

| Microgranules (taux d'enrobage 15%) | | Quantités (g) |
|---|---|---|
| MONTAGE | Neutres # 30 | 479,89 |
| | Sulfate de Morphine | 236,98 |
| | HPMC 603 | 83,13 |
| ENROBAGE | éthylcellulose | 400,00 |
| | *éq. masse sèche* | *120, 00* |
| | Triéthylcitrate | 29,00 |
| | Talc Pharma | 0,00 |
| SUR-ENROBAGE | HPMC 603 | 203,00 |
| | Talc Pharma | 203,00 |

| Bilan global | % |
|---|---|
| Neutres # 30 | 35,4 |
| Sulfate de Morphine | 17,5 |
| HPMC 603 | 21,1 |
| éthylcellulose | 8,9 |
| Triéthylcitrate | 2,1 |
| Talc Pharma | 14,9 |

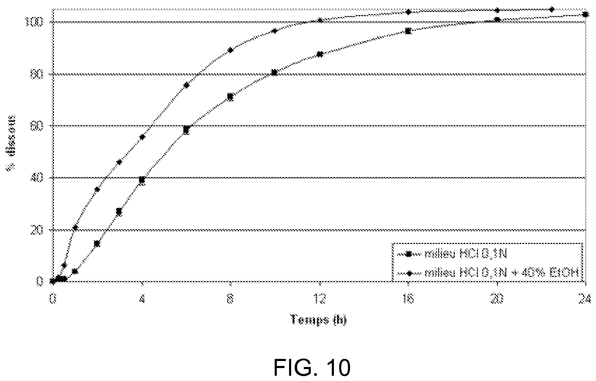

Les profils de dissolution obtenus dans l'HCl 0,1 N et le mélange HCl 0,1 N / Ethanol absolu concentré à 40 % (v/v) en éthanol absolu pour les microgranules sans couche de pré-montage, présentant un taux d'enrobage de 15% et contenant du talc en dehors de la couche d'enrobage sont consignés sur la figure 10. L'écart des pourcentages d'actif libéré au bout de 2 heures dans l'HCl 0,1 N par rapport aux milieux acido-alcoolique est de 21,1 %, soit supérieur à 15%, ce qui démontre que ces microgranules ne sont pas résistants à l'alcool.

### Contre-exemple 11 : Microgranules de diltiazem à libération prolongée (taux d'enrobage de 20%, exprimé en masse sèche de polymère d'enrobage par rapport à la masse de microgranules avant enrobage) ne contenant pas de talc dans la couche d'enrobage.

Les méthodes de préparation, de dosage et de dissolution des microgranules sont identiques à l'exemple 1, à l'exception de leur composition centésimale récapitulée dans le tableau suivant :

| Composition | % |
|---|---|
| Montage : | |
| Supports neutres de cellulose | 64,7 |
| Diltiazem HCl | 16,2 |
| PVP K30 | 2,0 |
| Enrobage : | |
| éthylcellulose | 13,4 |
| Triéthylcitrate | 3,2 |
| Aérosil® R972 | 0,5 |

L'écart des pourcentages d'actif libéré au bout de 2 heures dans l'HCl 0,1 N par rapport aux milieux acido-alcoolique (mélange HCl 0,1 N / Ethanol absolu concentré à 10, 20 et 40 % (v/v) en éthanol absolu) est indiqué dans le tableau suivant :

| | Ethanol 10 % | Ethanol 20 % | Ethanol 40% |
|---|---|---|---|
| Ecart à 2h (0-X% d'éthanol) | -20,2 % | 10,8 % | 53,3 % |
| Résistance à l'alcool (si écart <15%) | Oui | Oui | non |

## Revendications

1. Utilisation d'une charge inerte dans l'enrobage de microgranules à libération prolongée comprenant du centre vers la périphérie :
- un support neutre,
- au moins une couche de montage comprenant au moins un principe actif et un agent liant pharmaceutiquement acceptable; et
- au moins une couche d'enrobage à libération prolongée comprenant un polymère d'enrobage hydrophobe choisi parmi les dérivés non hydrosolubles de la cellulose pour conférer auxdites microgranules une résistance à l'alcool, **caractérisée en ce que** la quantité de charge inerte dans l'enrobage à libération prolongée des microgranules est d'au moins 20% de talc par rapport à la masse sèche du polymère d'enrobage hydrophobe, et **caractérisée en ce que** lesdites microgranules sont contenues dans une forme pharmaceutique orale à libération prolongée d'au moins un principe actif à administration journalière unique.

2. Utilisation selon la revendication 1, **caractérisée en ce que** le polymère d'enrobage hydrophobe desdits microgranules est choisi dans le groupe constitué par l'éthylcellulose, l'acétate butyrate de cellulose, l'acétate de cellulose et leurs mélanges.

3. Utilisation selon l'une quelconque des revendications 1 à 2, **caractérisée en ce que** la quantité de polymère d'enrobage hydrophobe desdits microgranules est comprise entre 30% à 80%, de préférence de 50% à 80%, de la masse sèche de ladite couche d'enrobage.

4. Utilisation selon l'une quelconque des revendications 1 à 3, **caractérisée en ce que** l'agent liant pharmaceutiquement acceptable desdits microgranules est choisi parmi les dérivés de la cellulose tels que l'HPMC, l'hydroxypropylcellulose, l'hydroxyéthylcellulose, les dérivés de la polyvinylpyrrolidone, et également les dérivés du polyéthylene glycol, des dérivés vinyliques tels que le polyvinylalcool et leurs mélanges.

5. Utilisation selon l'une quelconque des revendications 1 à 4, **caractérisée en ce que** lesdits microgranules comprennent un plastifiant dans la couche d'enrobage.

6. Utilisation selon l'une quelconque des revendications 1 à 5, **caractérisée en ce que** lesdits microgranules comprennent un agent tensio-actif dans la couche d'enrobage.

7. Utilisation selon l'une quelconque des revendications 1 à 6, **caractérisée en ce que** le principe actif est choisi parmi les hormones, les principes actifs agissant sur le système nerveux central, les principes actifs agissant sur le système cardiovasculaire, les antibiotiques, les anti-viraux et les analgésiques.

8. Utilisation selon la revendication 7, **caractérisée en ce que** le principe actif est choisi parmi les analgésiques et notamment les analgésiques non opiacés, opiacés faibles, opioïdes mixtes, morphiniques ou spasmodiques, notamment l'hydrocodone, l'hydromorphone, la morphine, l'oxycodone, l'oxymorphone, le tramadol et la gabapentine.

9. Utilisation selon les revendications 7 et 8, **caractérisée en ce que** le principe actif est la morphine.

10. Utilisation selon l'une quelconque des revendications 1 à 9 **caractérisée en ce que** lesdits microgranules comportent au moins une couche de pré-montage entre le support neutre et la couche de montage, ladite couche de pré-montage comprenant au moins un polymère hydrophobe, une charge inerte, optionnellement un plastifiant et/ou un agent tensio-actif.

11. Utilisation selon l'une quelconque des revendications 1 à 10, **caractérisée en ce que** le plastifiant desdits microgranules est choisi parmi : les glycérides acétylés, glycéryl-mono- stéarate, glycéryl-thacétate, glycéryl-tributyrate, les phtalates, dibutylphtalate, diéthylphtalate, diméthylphtalate, dioctylphtalate, les citrates, acétyltributylcitrate, acétylthéthylcitrate, tributylcitrate, triéthylcitrate, les sébacates, diéthylsébacate, dibutylsébacate, les adipates, les azélates, les benzoates, le chlorobutanoles polyéthylène glycols, les huiles végétales, les fumarates, le diéthylfumarate, les malates, le diéthylmalate, les oxalates, diéthyloxalate, les succinates ; le dibutylsuccinate, les butyrates, les esters de l'alcool cétylique, les malonates, le diéthylmalonate, l'huile de ricin, et leurs mélanges.

## Patentansprüche

1. Verwendung einer inerten Charge in der Ummantelung von Mikrogranulaten mit verlängerter Freisetzung, umfassend vom Zentrum zum Umfang:
- ein neutrales Substrat,
- mindestens eine Montageschicht, die mindestens ein aktives Prinzip und ein pharmazeutisch akzeptables Bindemittel umfasst, und
- mindestens eine Mantelschicht mit verlängerter Freisetzung, umfassend ein hydrophobes Mantelpolymer, das aus den nicht wasserlöslichen Derivaten der Zellulose ausgewählt ist, um den Mikrogranulaten eine Widerstandsfähigkeit gegenüber Alkohol zu verleihen, **dadurch gekennzeichnet, dass** die Menge inerter Charge in der Ummantelung mit verlängerter Freisetzung der Mikrogranulate mindestens 20 % Talk im Verhältnis zur Trockenmasse des hydrophoben Mantelpolymers beträgt, und **gekennzeichnet dadurch, dass** die Mikroganulate in einer oralen pharmazeutischen Form mit verlängerter Freisetzung mindestens eines aktiven Prinzips zur einzigen täglichen Freisetzung enthalten sind.

2. Verwendung nach Anspruch 1, **dadurch gekennzeichnet, dass** das hydrophobe Mantelpolymer der Mikrogranulate aus der Gruppe ausgewählt ist, die von der Ethylcellulose, dem Celluloseacetatbutyrat, dem Celluloseacetat und ihren Gemischen gebildet ist.

3. Verwendung nach einem der Ansprüche 1 bis 2, **dadurch gekennzeichnet, dass** die Menge hydrophoben Mantelpolymers der Mikrogranulate zwischen 30 % bis 80 % inklusive, vorzugsweise von 50 % bis 80 %, der Trockenmasse der Mantelschicht beträgt.

4. Verwendung nach einem der Ansprüche 1 bis 3, **dadurch gekennzeichnet, dass** das pharmazeutisch akzeptable Bindemittel der Mikrogranulate aus den Derivaten der Cellulose wie dem HPMC, der Hydroxypropylcellulose, der Hydroxyethylcellulose, den Derivaten des Polyvinylpyrrolidons und ebenfalls den Derivaten des Polyethylenglycols, den Vinylderivaten wie dem Polyvinylalkohol und ihren Gemischen ausgewählt ist.

5. Verwendung nach einem der Ansprüche 1 bis 4, **dadurch gekennzeichnet, dass** die Mikrogranulate einen Weichmacher in der Mantelschicht umfassen.

6. Verwendung nach einem der Ansprüche 1 bis 5, **dadurch gekennzeichnet, dass** die Mikrogranulate ein Tensid in der Mantelschicht umfassen.

7. Verwendung nach einem der Ansprüche 1 bis 6, **dadurch gekennzeichnet, dass** das aktive Prinzip aus den Hormonen, den auf das zentrale Nervensystem wirkenden aktiven Prinzipien, den auf das Herz-Kreislauf-System wirkenden aktiven Prinzipien, den Antibiotika, den antiviralen Mitteln und den Analgetika ausgewählt ist.

8. Verwendung nach Anspruch 7, **dadurch gekennzeichnet, dass** das aktive Prinzip aus den Analgetika ausgewählt ist und insbesondere aus den nicht opiumhaltigen, schwach opiumhaltigen, gemischt opiumhaltigen, morphinen oder spasmodischen Analgetika, insbesondere dem Hydrocodon, dem Hydromorphon, dem Morphin, dem Oxycodon, dem Oxymorphon, dem Tramadol, dem Gabapentin und ihren Derivaten ausgewählt ist.

9. Verwendung nach den Ansprüchen 7 und 8, **dadurch gekennzeichnet, dass** das aktive Prinzip das Morphin und seine Derivate ist.

10. Verwendung nach einem der Ansprüche 1 bis 9, **dadurch gekennzeichnet, dass** die Mikrogranulate mindestens eine Vormontageschicht zwischen dem neutralen Substrat und der Montageschicht aufweisen, wobei die Vormontageschicht mindestens ein hydrophobes Polymer, eine inerte Charge, optional einen Weichmacher und/oder ein Tensid umfasst.

11. Verwendung nach einem der Ansprüche 1 bis 10, **dadurch gekennzeichnet, dass** der Weichmacher der Mikrogranulate ausgewählt ist aus: den acetylierten Glyceriden, Glycerylmonostearat, Glyceryltriacetat, Glyceryltributyrat, den Phtalaten, Dibutylphtalat, Diethylphtalat, Dimethylphtalat, Dioctylphtalat, den Citraten, Acetyltributylcitrat, Acetylthethylcitrat, Tributylcitrat, Triethylcitrat, den Sebacaten, Diethylsebacat, Dibutylsebacat, den Adipaten, den Azelaten, den Benzoaten, dem Chlorobutanol-Polyethylenglycol, den Pflanzenölen, den Fumaraten, dem Diethylfumarat, den Malaten, dem Diethylmalat, den Oxalaten, Diethyloxalat, den Succinaten; den Dibutylsuccinat, den Butyraten, den Estern des Cetylalkohols, den Malonaten, dem Diethylmalonat, dem Ricinöl und ihren Gemischen.

## Claims

1. Use of an inert load in the coating of extended release microgranules comprising from the center toward the periphery:
- a neutral carrier,
- at least one mounting layer comprising at least one active ingredient and a pharmaceutically acceptable binder; and
- at least one extended-release coating layer comprising a hydrophobic coating polymer selected from non-water soluble cellulose derivatives, to confer on said microgranules a resistance to alcohol, **characterised in that** the quantity of the inert load in the extended release coating of the microgranules is at least 20% of talc in relation to the dry weight of the hydrophobic coating polymer, and **characterized in that** said microgranules are contained in an oral pharmaceutical form with extended release of at least one active ingredient for a single daily administration.

2. Use according to claim 1, **characterized in that** the hydrophobic coating polymer of said microgranules is selected from the group comprised of ethylcellulose, cellulose acetate butyrate, cellulose acetate and mixtures thereof.

3. Use according to any one of claims 1 to 2, **characterized in that** the quantity of the hydrophobic coating polymer of said microgranules is between 30% and 80%, preferably from 50% to 80%, of the dry weight of said coating layer.

4. Use according to any one of claims 1 to 3, **characterized in that** the pharmaceutically acceptable binder of said microgranules is selected from cellulose derivatives such as HPMC, hydroxypropylcellulose, hydroxyethylcellulose, polyvinylpyrrolidone derivatives, and also polyethylene glycol derivatives, vinyl derivatives such as polyvinyl alcohol and mixtures thereof.

5. Use according to any one of claims 1 to 4, **characterized in that** said microgranules comprise a plasticizer in the coating layer.

6. Use according to any one of claims 1 to 5, **characterized in that** said microgranules comprise a surfactant in the coating layer.

7. Use according to any one of claims 1 to 6, **characterized in that** the active ingredient is selected from hormones, active ingredients that act on the central nervous system, active ingredients that act on the cardiovascular system, antibiotics, antivirals and analgesics.

8. Use according to claim 7, **characterized in that** the active ingredient is selected from analgesics and notably non-opiate, weak opiate, mixed opioid, morphine or spasmodic analgesics, notably hydrocodone, hydromorphone, morphine, oxycodone, oxymorphone, tramadol and gabapentin.

9. Use according to claims 7 and 8, **characterized in that** the active ingredient is morphine.

10. Use according to any one of claims 1 to 9, **characterized in that** said microgranules comprise at least one pre-mounting layer between the neutral carrier and the mounting layer, said pre-mounting layer comprising at least one hydrophobic polymer, an inert load, optionally a plasticizer and/or a surfactant.

11. Use according to any one of claims 1 to 10, **characterized in that** the plasticizer of said microgranules is selected from: acetylated glycerides, glyceryl monostearate, glyceryl triacetate, glyceryl tributyrate, phthalates, dibutyl phthalate, diethyl phthalate, dimethyl phthalate, dioctyl phthalate, citrates, acetyl tributyl citrate, acetyl triethyl citrate, tributyl citrate, triethyl citrate, sebacates, diethyl sebacate, dibutyl sebacate, adipates, azelates, benzoates, chlorobutanols, polyethylene glycols, plant oils, fumarates, diethyl fumarate, malates, diethyl malate, oxalates, diethyl oxalate, succinates, dibutyl succinate, butyrates, cetyl alcohol esters, malonates, diethyl malonate, castor oil and mixtures thereof.
